# EUROPEAN PATENT APPLICATION

(11) **EP 4 349 949 A1**
(43) Date of publication of application: **10.04.2024**
(21) Application number: 22199856.0
(22) Date of filing: 05.10.2022
(51) Int. Cl.: C11D 3/33, C07C 227/42, C11D 11/00

(54) **PROCESS FOR MAKING A SOLID ALKALI METAL SALT OF AN AMINOCARBOXYLATE COMPLEXING AGENT**

(71) Applicant: BASF SE, 67056 Ludwigshafen am Rhein (DE)
(72) Inventor: The designation of the inventor has not yet been filed
(74) Representative: BASF IP Association

(57) **Abstract**

The present invention relates to a process for making a solid alkali metal salt (A) of an aminocarboxylate complexing agent.

## Description

The present invention relates to a process for making a solid alkali metal salt (A) of an aminocarboxylate complexing agent.

Complexing agents such as methyl glycine diacetic acid (MGDA) and glutamic acid diacetic acid (GLDA) and their respective alkali metal salts are useful sequestrants for alkaline earth metal ions such as Ca²⁺ and Mg²⁺. For that reason, they are recommended and used for various purposes such as laundry detergents and for automatic dishwashing (ADW) formulations, in particular for so-called phosphate-free laundry detergents and phosphate-free ADW formulations. For shipping such complexing agents, in most cases either solids such as granules are being applied or aqueous solutions.

Granules and powders have the advantage of being essentially water-free. That means that in case of shipping, no water has to be shipped, and costs for extra weight can be avoided.

Many industrial users prefer to use chelating agents in the form of granules or powders which may contain, in addition to the at least one chelating agent, at least one additional compound, in particular polymers. Some customers also wish to incorporate higher amounts of additional compounds, e. g. polymers, into the granules or powders containing at least one chelating agent.

Methods for providing granules or powders of chelating agents, also in combination with other compounds like polymers, have been described in the art, for example in WO 2015/121170 A1.

However, conventional processes to prepare chelating agents, e. g. aminocarboxylate chelating agents, in solid form, are associated with disadvantages. For example, spray granulation processes for solidifying aminocarboxylate chelating agents consume a lot of energy. Furthermore, a considerable amount of the solid products obtained from a spray granulation process tend to be in monoclinic space group, which show an unwanted yellowing in the presence of percarbonates ("percarbonate stability test"), which are frequently found in cleaning products also containing aminocarboxylate chelating agents, and show a stronger tendency to adsorb water (higher hygroscopicity), which is undesirable.

Thus, one objective of the present invention was to overcome the problems and disadvantages mentioned above. In particular, it was an objective of the present invention to provide a process for making a solid alkali metal salt (A) of an aminocarboxylate complexing agent which consumes less energy than traditional solidication processes, and/or which leads to an increased content of aminocarboxylate chelating agents in orthorhombic space group.

Furthermore, it was another objective of the present invention to provide solid alkali metal salts (A) of an aminocarboxylate complexing agent which are mainly in orthorhombic space group. Besides, it was another objective of the present invention to provide solid alkali metal salts (A) of an aminocarboxylate complexing agent which show a decreased yellowing in the presence of percarbonates, and/or which show a lower hygroscopicity.

Surprisingly, it was found that a process which involves a drying device comprising at least one rotably mounted heated surface (e. g. disc or cylinder) for receiving an aqueous solution or slurry containing an aminocarboxylate complexing agent leads to unexpected improved results, both in the process itself (reduced energy consumption) and the product (improved product quality).

One object of the present invention is therefore a process for making a solid alkali metal salt (A) of an aminocarboxylate complexing agent, said process comprising the steps of
a) providing a 40 to 80% by weight aqueous solution or slurry, preferably slurry, comprising a salt (A),
b) introducing the slurry or solution from step (a) into a drying device comprising at least one rotably mounted heated surface for receiving the solution or slurry,
c) heating said surface to a temperature in the range of from 50° to 250°, preferably in the range from 50° to 220° C, wherein the heating has the effect that the solution or slurry on the rotably mounted surface evaporates and a solid salt (A) remains, and).
d) removing said solid salt (A).

In one embodiment of the inventive process, step c) is performed after step b), i. e. heating of the rotably mounted surface to the temperatures mentioned in step c) is done after the introduction of the slurry or solution from step (a) into the drying device.

In another preferred embodiment of the inventive process, heating of the rotably mounted surface to the temperatures mentioned in step c) is done during or, preferably, before introducing the slurry or solution from step (a) into the drying device.

A further object of the present invention is the use of a drying device comprising a rotably mounted surface for receiving a solution or slurry, for drying a 40 to 80% by weight aqueous solution or slurry comprising a solid alkali metal salt (A) of an aminocarboxylate complexing agent.

Another object of the present invention is also a solid alkali metal salt (A) of a mixture of enantiomers of the trisodium salt of MGDA wherein said solid comprises at least 60% by weight of a crystalline material whose lattice has an orthorhombic space group, preferably obtained or obtainable by said inventive process.

The percentages of orthorhombic salt were determined by X-ray diffraction. The X ray powder diffractometer measurements were carried out on a D8 Advance^{®} diffractometer from Bruker AXS (Karlsruhe). In reflection with Cu-K α radiation was measured with a variable diaphragm adjustment on the primary side and on the secondary side. The measurement range was 2° to 80° 2-theta, the step width 0.01° and the measurement time per angle step 3.6 seconds. Based on the software TOPAS from Bruker optics, the relative amounts of the polymorphic form were determined.

A further object of the present invention is the use of a solid alkali metal salt (A) of an aminocarboxylate complexing agent, preferably obtained or obtainable by the inventive process, for the manufacture of cleaning agents, preferably selected from dishwashing and laundry detergents, more preferably dishwashing detergents, particularly automatic dishwashing detergents.

Another object of the present invention is a cleaning agent, preferably selected from dishwashing and laundry detergents, more preferably dishwashing detergents, particularly automatic dishwashing detergents, comprising an aminocarboxylate complexing agent, preferably obtained or obtainable by the inventive, and optionally an antimicrobial agent selected from the group consisting of 2-phenoxyethanol; preferably comprising said antimicrobial agent in an amount ranging from 2ppm to 5% by weight of the composition; more preferably comprising 0.1 to 2% of phenoxyethanol.

In an embodiment of the inventive process, the rotably mounted surface in the drying device is heated by a heating fluid, inductively and/or by hot steam.

In a preferred embodiment of the inventive process, said aminocarboxylate complexing agent is selected from methylglycine diacetic acid (MGDA), glutamic acid diacetic acid (GLDA) and/or ethylenediamine-N,N'-disuccinic acid (EDDS). MGDA is particularly preferred.

In another embodiment of the inventive process, salt (A) is selected from compounds according to general formula (I)

[CH₃-CH(COO)-N(CH₂-COO)₂]M₃₋ₓHₓ (I)

wherein
M is selected from alkali metal cations, same or different, and
x in formula is in the range of from zero to 1.0.

In a further embodiment of the inventive process, methylglycine diacetate (MGDA) alkali metal salt (A) is selected from the racemic mixture and mixtures of the enantiomers with predominantly the L-enantiomer with an ee value in the range of from 0.1 to 35%.

In one embodiment of the inventive process, salt (A) withdrawn in step (d) has a moisture content in the range of from 1 to 30% by weight, preferably 1 to 20% by weight, relative to the total weight of salt (A).

The dry content was determined by drying the said sample for 60min at 200°C on a drying balance.

In another embodiment of the inventive process, the solution or slurry provided in step (a) contains at least 0.1 weight% of a further organic or inorganic compound or mixtures thereof.

In a further embodiment of the inventive process, the solution or slurry provided in step (a) contains at least 0.1 weight% of a (co)polymer (B) selected from (co)polymers of (meth)acrylic acid and polyethylenimines, non-substituted or substituted with alkoxy groups or CH₂COOH groups that may be neutralized with alkali metal.

In another embodiment of the inventive process, a sieving step and/or a milling step and/or a compacting step is performed subsequently to step (d), and/or wherein solid alkali metal salt (A) is obtained in the form of a flake, a powder and/or crystal.

In a preferred embodiment of the inventive process, the drying device is a disc dryer. For example, a device called "Scheibentrockner CDry" provided by Allgaier Process Technology GmbH, Germany, may be used.

In one embodiment of the inventive process, the pressure in the drying device is in the range from 100 to 1100 mbar abs, preferably 100 to 600 mbar abs, more preferably 300 to 600 mbar abs.

### Applications

Inventive solid alkali metal salts (A) of an aminocarboxylate complexing agent, e. g. powders and granules, exhibit overall advantageous properties including but not limited to an excellent yellowing behavior, especially in the presence of bleaching agents. They are therefore excellently suitable for the manufacture of cleaning agents that contain at least one bleaching agent, such cleaning agent hereinafter also being referred to as bleach. In particular inventive solid compositions are suitable for the manufacture cleaning agent for fibers or hard surfaces wherein said cleaning agent contains at least one peroxy compound.

Inventive solid compositions (e. g. powders) may easily be converted into compactates and into agglomerates.

Another aspect of the present invention is therefore the use of solid alkali metal salt (A) of an aminocarboxylate complexing agent for the manufacture of a cleaning agent that contains at least one bleaching agent, and in particular for the manufacture of cleaning agent for fibers or hard surfaces, wherein said cleaning agent contains at least one peroxy compound. Another aspect of the present invention is a process for making at a cleaning agent by combining at least one inventive solid alkali metal salt (A) of an aminocarboxylate complexing agent with at least one bleaching agent, preferably at least one peroxy compound. Another aspect of the present invention is a cleaning agent, hereinafter also being referred to as inventive cleaning agent. Inventive cleaning agents contain at least one bleaching agent and at least one inventive solid alkali metal salt (A) of an aminocarboxylate complexing agent (e. g. powder). Inventive cleaning agents show a reduced tendency for yellowing and therefore have an extended shelve-life.

Examples of suitable peroxy compounds are sodium perborate, anhydrous or for example as monohydrate or as tetrahydrate or so-called dihydrate, sodium percarbonate, anhydrous or, for example, as monohydrate, hydrogen peroxide, persulfates, organic peracids such as peroxylauric acid, peroxystearic acid, peroxy-α-naphthoic acid, 1,12-diperoxydodecanedioic acid, perbenzoic acid, peroxylauric acid, 1,9-diperoxyazelaic acid, diperoxyisophthalic acid, in each case as free acid or as alkali metal salt, in particular as sodium salt, also sulfonylperoxy acids and cationic peroxy acids.

In a preferred embodiment, peroxy compound is selected from inorganic percarbonates, persulfates and perborates. Examples of sodium percarbonates are 2 Na₂CO₃·3 H₂O₂. Examples of sodium perborate are (Na₂[B(OH)₂(O₂)]₂), sometimes written as NaBO₂·O₂·3H₂O instead. Most preferred peroxy compound is sodium percarbonate

The term "cleaning agents" includes compositions for dishwashing, especially hand dishwash and automatic dishwashing and ware-washing, and compositions for hard surface cleaning such as, but not limited to compositions for bathroom cleaning, kitchen cleaning, floor cleaning, descaling of pipes, window cleaning, car cleaning including truck cleaning, furthermore, open plant cleaning, cleaning-in-place, metal cleaning, disinfectant cleaning, farm cleaning, high pressure cleaning, and in addition, laundry detergent compositions.

Such cleaning agents may be liquids, gels or preferably solids at ambient temperature, solids cleaning agents being preferred. They may be in the form of a powder or in the form of a unit dose, for example as a tablet or pouch.

In one embodiment of the present invention, inventive cleaning agents may contain
in the range of from 2 to 50 % by weight of inventive solid alkali metal salt (A) of an aminocarboxylate complexing agent and
in the range of from 0.5 to 15 % by weight of bleach.

Percentages are based on the solids content of the respective inventive cleaning agent.

Inventive solid alkali metal salts (A) of an aminocarboxylate complexing agent are excellently suited for the manufacture of laundry detergents or cleaners.

Inventive cleaning agents may contain further ingredients such as one or more surfactants that may be selected from non-ionic, zwitterionic, cationic, and anionic surfactants. Other ingredients that may be contained in inventive cleaning agents may be selected from bleach activators, bleach catalysts, corrosion inhibitors, sequestering agents other than chelating agent (A), enzymes, fragrances, dyestuffs, antifoams, and builders.

Particularly advantageous inventive cleaning agents may contain one or more complexing agents other than MGDA or GLDA. Advantageous detergent compositions for cleaners and advantageous laundry detergent compositions may contain one or more sequestrant (chelating agent) other than a mixture according to the present invention. Examples for sequestrants other than a mixture according to the present invention are IDS (iminodisuccinate), citrate, phosphonic acid derivatives, for example the disodium salt of hydroxyethane-1,1-diphosphonic acid ("HEDP"), and polymers with complexing groups like, for example, polyethyleneimine in which 20 to 90 mole-% of the N-atoms bear at least one CH₂COO⁻ group, and their respective alkali metal salts, especially their sodium salts, for example IDS-Na₄, and trisodium citrate, and phosphates such as STPP (sodium tripolyphosphate). Due to the fact that phosphates raise environmental concerns, it is preferred that advantageous inventive cleaning agents are free from phosphate. "Free from phosphate" should be understood in the context of the present invention, as meaning that the content of phosphate and polyphosphate is in sum in the range from 10 ppm to 0.2% by weight, determined by gravimetric methods and referring to the respective inventive cleaning agent.

Inventive cleaning agents may contain one or more surfactant, preferably one or more non-ionic surfactant.

Preferred non-ionic surfactants are alkoxylated alcohols, di- and multiblock copolymers of ethylene oxide and propylene oxide and reaction products of sorbitan with ethylene oxide or propylene oxide, alkyl polyglycosides (APG), hydroxyalkyl mixed ethers and amine oxides.

Preferred examples of alkoxylated alcohols and alkoxylated fatty alcohols are, for example, compounds of the general formula (II) 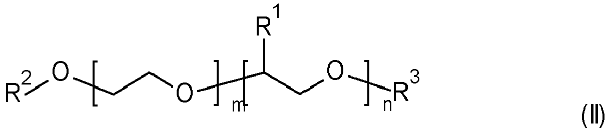 in which the variables are defined as follows:
- R¹: is identical or different and selected from hydrogen and linear C₁-C₁₀-alkyl, preferably in each case identical and ethyl and particularly preferably hydrogen or methyl,
- R²: is selected from C₈-C₂₂-alkyl, branched or linear, for example n-C₈H₁₇, n-C₁₀H₂₁, n-C₁₂H₂₅, n-C₁₄H₂₉, n-C₁₆H₃₃ or n-C₁₈H₃₇,
- R³: is selected from C₁-C₁₀-alkyl, methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl or isodecyl,
m and n are in the range from zero to 300, where the sum of n and m is at least one, preferably in the range of from 3 to 50. Preferably, m is in the range from 1 to 100 and n is in the range from 0 to 30.

In one embodiment, compounds of the general formula (II) may be block copolymers or random copolymers, preference being given to block copolymers.

Other preferred examples of alkoxylated alcohols are, for example, compounds of the general formula (III) 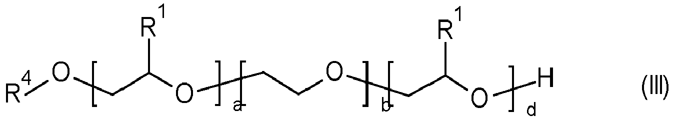 in which the variables are defined as follows:
- R¹: is identical or different and selected from hydrogen and linear C₁-C₀-alkyl, preferably identical in each case and ethyl and particularly preferably hydrogen or methyl,
- R⁴: is selected from C₆-C₂₀-alkyl, branched or linear, in particular n-C₈H₁₇, n-C₁₀H₂₁, n-C₁₂H₂₅, n-C₁₄H₂₉, n-C₁₆H₃₃, n-C₁₈H₃₇,
- a: is a number in the range from zero to 10, preferably from 1 to 6,
- b: is a number in the range from 1 to 80, preferably from 4 to 20,
- d: is a number in the range from zero to 50, preferably 4 to 25.

The sum a + b + d is preferably in the range of from 5 to 100, even more preferably in the range of from 9 to 50.

Preferred examples for hydroxyalkyl mixed ethers are compounds of the general formula (IV) 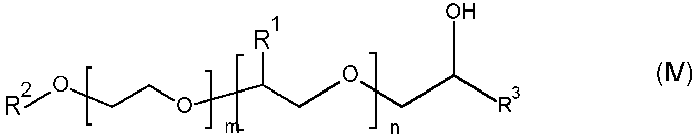 in which the variables are defined as follows:
- R¹: is identical or different and selected from hydrogen and linear C₁-C₁₀-alkyl, preferably in each case identical and ethyl and particularly preferably hydrogen or methyl,
- R²: is selected from C₈-C₂₂-alkyl, branched or linear, for example iso-C₁₁H₂₃, iso-C₁₃H₂₇, n-C₈H₁₇, n-C₁₀H₂₁, n-C₁₂H₂₅, n-C₁₄H₂₉, n-C₁₆H₃₃ or n-C₁₈H₃₇,
- R³: is selected from C₁-C₁₈-alkyl, methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl, isodecyl, n-dodecyl, n-tetradecyl, n-hexadecyl, and n-octadecyl.

The variables m and n are in the range from zero to 300, where the sum of n and m is at least one, preferably in the range of from 5 to 50. Preferably, m is in the range from 1 to 100 and n is in the range from 0 to 30.

Compounds of the general formula (II) and (III) may be block copolymers or random copolymers, preference being given to block copolymers.

Further suitable nonionic surfactants are selected from di- and multiblock copolymers, composed of ethylene oxide and propylene oxide. Further suitable nonionic surfactants are selected from ethoxylated or propoxylated sorbitan esters. Amine oxides or alkyl polyglycosides, especially linear C₄-C₁₆-alkyl polyglucosides and branched C₈-C₁₄-alkyl polyglycosides such as compounds of general average formula (V) are likewise suitable. 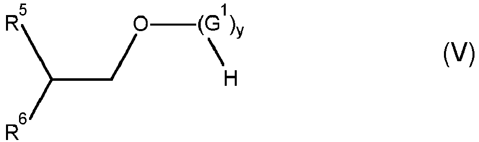 wherein the variables are defined as follows:
- R⁵: is C₁-C₄-alkyl, in particular ethyl, n-propyl or isopropyl,
- R⁶: is -(CH₂)₂-R⁵,
- G¹: is selected from monosaccharides with 4 to 6 carbon atoms, especially from glucose and xylose,
- y: in the range of from 1.1 to 4, y being an average number.

Further examples of non-ionic surfactants are compounds of general formula (VII) and (VIII) 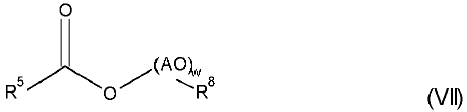 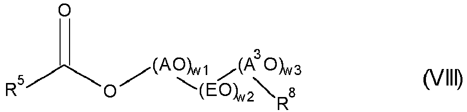
AO is selected from ethylene oxide, propylene oxide and butylene oxide,
EO is ethylene oxide, CH₂CH₂-O,
R⁸ selected from C₈-C₁₈-alkyl, branched or linear, and R⁵ is defined as above.
A³O is selected from propylene oxide and butylene oxide,
w is a number in the range of from 15 to 70, preferably 30 to 50,
w1 and w3 are numbers in the range of from 1 to 5, and
w2 is a number in the range of from 13 to 35.

An overview of suitable further nonionic surfactants can be found in EP-A 0 851 023 and in DE-A 198 19 187.

Mixtures of two or more different nonionic surfactants may also be present.

Other surfactants that may be present are selected from amphoteric (zwitterionic) surfactants and anionic surfactants and mixtures thereof.

Examples of amphoteric surfactants are those that bear a positive and a negative charge in the same molecule under use conditions. Preferred examples of amphoteric surfactants are so-called betaine-surfactants. Many examples of betaine-surfactants bear one quaternized nitrogen atom and one carboxylic acid group per molecule. A particularly preferred example of amphoteric surfactants is cocamidopropyl betaine (lauramidopropyl betaine).

Examples of amine oxide surfactants are compounds of the general formula (IX)

R⁷R⁸R⁹N→O (IX)

wherein R⁷, R⁸ and R⁹ are selected independently from each other from aliphatic, cycloaliphatic or C₂-C₄-alkylene C₁₀-C₂₀-alkylamido moieties. Preferably, R⁷ is selected from C₈-C₂₀-alkyl or C₂-C₄-alkylene C₁₀-C₂₀-alkylamido and R⁸ and R⁹ are both methyl.

A particularly preferred example is lauryl dimethyl aminoxide, sometimes also called lauramine oxide. A further particularly preferred example is cocamidylpropyl dimethylaminoxide, sometimes also called cocamidopropylamine oxide.

Examples of suitable anionic surfactants are alkali metal and ammonium salts of C₈-C₁₈-alkyl sulfates, of C₈-C₁₈-fatty alcohol polyether sulfates, of sulfuric acid half-esters of ethoxylated C₄-C₁₂-alkylphenols (ethoxylation: 1 to 50 mol of ethylene oxide/mol), C₁₂-C₁₈ sulfo fatty acid alkyl esters, for example of C₁₂-C₁₈ sulfo fatty acid methyl esters, furthermore of C₁₂-C₁₈-alkylsulfonic acids and of C₁₀-C₁₈-alkylarylsulfonic acids. Preference is given to the alkali metal salts of the aforementioned compounds, particularly preferably the sodium salts.

Further examples for suitable anionic surfactants are soaps, for example the sodium or potassium salts of stearoic acid, oleic acid, palmitic acid, ether carboxylates, and alkylether phosphates.

Preferably, laundry detergent compositions contain at least one anionic surfactant.

In one embodiment of the present invention, inventive cleaning agents that are determined to be used as laundry detergent compositions may contain 0.1 to 60 % by weight of at least one surfactant, selected from anionic surfactants, amphoteric surfactants and amine oxide surfactants. In one embodiment of the present invention, inventive cleaning agents that are determined to be used for hard surface cleaning may contain 0.1 to 60 % by weight of at least one surfactant, selected from anionic surfactants, amphoteric surfactants and amine oxide surfactants.

In a preferred embodiment, inventive cleaning agents do not contain any anionic detergent.

Inventive cleaning agents may comprise one or more bleach catalysts. Bleach catalysts can be selected from bleach-boosting transition metal salts or transition metal complexes such as, for example, manganese-, iron-, cobalt-, ruthenium- or molybdenum-salen complexes or carbonyl complexes. Manganese, iron, cobalt, ruthenium, molybdenum, titanium, vanadium and copper complexes with nitrogen-containing tripod ligands and also cobalt-, iron-, copper- and ruthenium-amine complexes can also be used as bleach catalysts.

Inventive cleaning agents may comprise one or more bleach activators, for example N-methyl-morpholinium-acetonitrile salts ("MMA salts"), trimethylammonium acetonitrile salts, N-acylimides such as, for example, N-nonanoylsuccinimide, 1,5-diacetyl-2,2-dioxohexahydro-1,3,5-triazine ("DADHT") or nitrile quats (trimethylammonium acetonitrile salts).

Further examples of suitable bleach activators are tetraacetylethylenediamine (TAED) and tetraacetylhexylenediamine.

Inventive cleaning agents may comprise one or more corrosion inhibitors. In the present case, this is to be understood as including those compounds which inhibit the corrosion of metal. Examples of suitable corrosion inhibitors are triazoles, in particular benzotriazoles, bisbenzotriazoles, aminotriazoles, alkylaminotriazoles, also phenol derivatives such as, for example, hydroquinone, pyrocatechol, hydroxyhydroquinone, gallic acid, phloroglucinol or pyrogallol.

In one embodiment of the present invention, inventive cleaning agents comprise in total in the range from 0.1 to 1.5% by weight of corrosion inhibitor.

Inventive cleaning agents may comprise one or more builders, selected from organic and inorganic builders. Examples of suitable inorganic builders are sodium sulfate or sodium carbonate or silicates, in particular sodium disilicate and sodium metasilicate, zeolites, sheet silicates, in particular those of the formula α-Na₂Si₂O₅, β-Na₂Si₂O₅, and δ-Na₂Si₂O₅, also fatty acid sulfonates, α-hydroxypropionic acid, alkali metal malonates, fatty acid sulfonates, alkyl and alkenyl disuccinates, tartaric acid diacetate, tartaric acid monoacetate, oxidized starch, and polymeric builders, for example polycarboxylates and polyaspartic acid.

Examples of organic builders are especially polymers and copolymers other such as (co)polymers (B) and include polymers and copolymers than (co)polymer (B), or one additional (co)polymer (B). In one embodiment of the present invention, organic builders are selected from polycarboxylates, for example alkali metal salts of (meth)acrylic acid homopolymers or (meth)acrylic acid copolymers, partially or completely neutralized with alkali.

Suitable comonomers for (meth)acrylic acid are monoethylenically unsaturated dicarboxylic acids such as maleic acid, fumaric acid, maleic anhydride, itaconic acid and citraconic acid. A suitable polymer is in particular polyacrylic acid, which preferably has an average molecular weight M_{w} in the range from 2000 to 40 000 g/mol, preferably 2000 to 10 000 g/mol, in particular 3000 to 8000 g/mol. Also of suitability are copolymeric polycarboxylates, in particular those of acrylic acid with methacrylic acid and of acrylic acid or methacrylic acid with maleic acid and/or fumaric acid, and in the same range of molecular weight.

It is also possible to use copolymers of at least one monomer from the group consisting of monoethylenically unsaturated C₃-C₁₀-mono- or C₄-C₁₀-dicarboxylic acids or anhydrides thereof, such as maleic acid, maleic anhydride, acrylic acid, methacrylic acid, fumaric acid, itaconic acid and citraconic acid, with at least one hydrophilic or hydrophobic monomer as listed below.

Suitable hydrophobic monomers are, for example, isobutene, diisobutene, butene, pentene, hexene and styrene, olefins with 10 or more carbon atoms or mixtures thereof, such as, for example, 1-decene, 1-dodecene, 1-tetradecene, 1-hexadecene, 1-octadecene, 1-eicosene, 1-docosene, 1-tetracosene and 1-hexacosene, C₂₂-α-olefin, a mixture of C₂₀-C₂₄-α-olefins and polyisobutene having on average 12 to 100 carbon atoms per molecule.

Suitable hydrophilic monomers are monomers with sulfonate or phosphonate groups, and also nonionic monomers with hydroxyl function or alkylene oxide groups. By way of example, mention may be made of: allyl alcohol, isoprenol, methoxypolyethylene glycol (meth)acrylate, methoxypolypropylene glycol (meth)acrylate, methoxypolybutylene glycol (meth)acrylate, methoxypoly(propylene oxide-co-ethylene oxide) (meth)acrylate, ethoxypolyethylene glycol (meth)acrylate, ethoxypolypropylene glycol (meth)acrylate, ethoxypolybutylene glycol (meth)acrylate and ethoxypoly(propylene oxide-co-ethylene oxide) (meth)acrylate. Polyalkylene glycols here may comprise 3 to 50, in particular 5 to 40 and especially 10 to 30 alkylene oxide units per molecule. Particularly preferred sulfonic-acid-group-containing monomers here are 1-acrylamido-1-propanesulfonic acid, 2-acrylamido-2-propanesulfonic acid, 2-acrylamido-2-methylpropanesulfonic acid, 2-methacrylamido-2-methylpropanesulfonic acid, 3-methacrylamido-2-hydroxypropanesulfonic acid, allylsulfonic acid, methallylsulfonic acid, allyloxybenzenesulfonic acid, methal-lyloxybenzenesulfonic acid, 2-hydroxy-3-(2-propenyloxy)propanesulfonic acid, 2-methyl-2-propene-1-sulfonic acid, styrenesulfonic acid, vinylsulfonic acid, 3-sulfopropyl acrylate, 2-sulfoethyl methacrylate, 3-sulfopropyl methacrylate, sulfomethacrylamide, sulfomethylmethacrylamide, and salts of said acids, such as sodium, potassium or ammonium salts thereof.

Particularly preferred phosphonate-group-containing monomers are vinylphosphonic acid and its salts.

A further example of builders is carboxymethyl inulin.

Moreover, amphoteric polymers can also be used as builders.

Inventive cleaning agents may comprise, for example, in the range from in total 10 to 70% by weight, preferably from in total 10 to 50% by weight, more preferably up to 20% by weight, of builder.

In one embodiment of the present invention, inventive cleaning agents according to the invention may comprise one or more co-builders.

Inventive cleaning agents may comprise one or more antifoams, selected for example from silicone oils and paraffin oils.

In one embodiment of the present invention, inventive cleaning agents comprise in total in the range from 0.05 to 0.5% by weight of antifoam.

Inventive cleaning agents may comprise one or more enzymes. Examples of enzymes are lipases, hydrolases, amylases, proteases, cellulases, esterases, pectinases, lactases and peroxidases.

In one embodiment of the present invention, inventive cleaning agents may comprise, for example, up to 5% by weight of enzyme, preference being given to 0.1 to 3% by weight. Said enzyme may be stabilized, for example with the sodium salt of at least one C₁-C₃-carboxylic acid or C₄-C₁₀-dicarboxylic acid. Preferred are formates, acetates, adipates, and succinates.

In one embodiment of the present invention, inventive cleaning agents may comprise at least one zinc salt. Zinc salts can be selected from water-soluble and water-insoluble zinc salts. In this connection, within the context of the present invention, water-insoluble is used to refer to those zinc salts which, in distilled water at 25°C, have a solubility of 0.1 g/l or less. Zinc salts which have a higher solubility in water are accordingly referred to within the context of the present invention as water-soluble zinc salts.

In one embodiment of the present invention, zinc salt is selected from zinc benzoate, zinc gluconate, zinc lactate, zinc formate, ZnCl₂, ZnSO₄, zinc acetate, zinc citrate, Zn(NO₃)₂, Zn(CH₃SO₃)₂ and zinc gallate, preferably ZnCl₂, ZnSO₄, zinc acetate, zinc citrate, Zn(NO₃)₂, Zn(CH₃SO₃)₂ and zinc gallate.

In another embodiment of the present invention, zinc salt is selected from ZnO, ZnO·aq, Zn(OH)₂ and ZnCO₃. Preference is given to ZnO·aq.

In one embodiment of the present invention, zinc salt is selected from zinc oxides with an average particle diameter (weight-average) in the range from 10 nm to 100 µm.

The cation in zinc salt can be present in complexed form, for example complexed with ammonia ligands or water ligands, and in particular be present in hydrated form. To simplify the notation, within the context of the present invention, ligands are generally omitted if they are water ligands.

Depending on how the pH value of mixture according to the invention is adjusted, zinc salt can change. Thus, it is for example possible to use zinc acetate or ZnCl₂ for preparing formulation according to the invention, but this converts at a pH of 8 or 9 in an aqueous environment to ZnO, Zn(OH)₂ or ZnO·aq, which can be present in non-complexed or in complexed form.

Zinc salt may be present in those inventive cleaning agents that are solid at room temperature. In such inventive cleaning agents zinc salts are preferably present in the form of particles which have for example an average diameter (number-average) in the range from 10 nm to 100 µm, preferably 100 nm to 5 µm, determined for example by X-ray scattering.

Zinc salt may be present in those inventive cleaning agents that are liquid at room temperature. In such inventive cleaning agents zinc salts are preferably present in dissolved or in solid or in colloidal form.

In one embodiment of the present invention, inventive cleaning agents comprise in total in the range from 0.05 to 0.4% by weight of zinc salt, based in each case on the dry content of the cleaning agent in question.

Here, the fraction of zinc salt is given as zinc or zinc ions. From this, it is possible to calculate the counterion fraction.

In one embodiment of the present invention, inventive cleaning agents are free from heavy metals apart from zinc compounds. Within the context of the present, this may be understood as meaning that inventive cleaning agents are free from those heavy metal compounds which do not act as bleach catalysts, in particular of compounds of iron and of bismuth. Within the context of the present invention, "free from" in connection with heavy metal compounds is to be understood as meaning that the content of heavy metal compounds which do not act as bleach catalysts is in sum in the range from 0 to 100 ppm, determined by the leach method and based on the dry content. Preferably, inventive cleaning agents has, apart from zinc, a heavy metal content below 0.05 ppm, based on the dry content of the formulation in question. The fraction of zinc is thus not included.

Within the context of the present invention, "heavy metals" are deemed to be all metals with a specific density of at least 6 g/cm³ with the exception of zinc. In particular, the heavy metals are metals such as bismuth, iron, copper, lead, tin, nickel, cadmium and chromium.

Preferably, inventive cleaning agents comprise no measurable fractions of bismuth compounds, for example less than 1 ppm.

Inventive cleaning agents are excellent for cleaning hard surfaces and fibres. For example, they may be used in dishwashing applications, preferably automatic dishwashing applications.

In one embodiment of the present invention, inventive cleaning agents comprise one or more further ingredient such as fragrances, dyestuffs, organic solvents, buffers, disintegrants for tablets ("tabs"), and/or acids such as methylsulfonic acid.

From inventive solid compositions, e. g. granules or powders, examplary detergent compositions for automatic dishwashing detergents can be formulated by mixing the respective components according to the following Table F.

**Table F: Example detergent compositions for automatic dishwashing**

| All amounts in g/sample | ADW.1 | ADW.2 | ADW.3 |
|---|---|---|---|
| Inventive granule (solid alkali metal salt) | 30 | 22.5 | 15 |
| Protease | 2.5 | 2.5 | 2.5 |
| Amylase | 1 | 1 | 1 |
| n-C₁₈H₃₇-O(CH₂CH₂O)₉H | 5 | 5 | 5 |
| Sodium percarbonate | 10.5 | 10.5 | 10.5 |
| TAED | 4 | 4 | 4 |
| Na₂CO₃ | 19.5 | 19.5 | 19.5 |
| Sodium citrate dihydrate | 15 | 22.5 | 30 |
| HEDP | 0.5 | 0.5 | 0.5 |
| ethoxylated polyethylenimine, 20 EO/NH group, Mₙ: 30,000 g/mol | optionally: 0.1 | optionally: 0.1 | optionally: 0.1 |

Laundry detergents according to the invention are useful for laundering any type of laundry, and any type of fibres. Fibres can be of natural or synthetic origin, or they can be mixtures of natural of natural and synthetic fibres. Examples of fibers of natural origin are cotton and wool. Examples for fibers of synthetic origin are polyurethane fibers such as Spandex^{®} or Lycra^{®}, polyester fibers, or polyamide fibers. Fibers may be single fibers or parts of textiles such as knitwear, wovens, or nonwovens.

Another aspect of the present invention is a process for making tablets for automatic dishwashing from an inventive solid alkali metal salt (A) of an aminocarboxylate complexing agent, e. g. a powder or granule, wherein said granule or powder is selected from inventive granules and inventive powders, respectively. Said process is hereinafter also referred to as pelletizing process according to the invention.

Inventive tablets are preferably made with the help of a machine, for example a tablet press. The pelletizing process according to the invention can be carried out by mixing an inventive solid alkali metal salt (A) of an aminocarboxylate complexing agent, e. g. powder, with at least one non-ionic surfactant and optionally one or more further substance and then compressing the mixture to give tablets. Examples of suitable non-ionic surfactants and further substances such as builders, enzymes are listed above. Particularly preferred examples of non-ionic surfactants are hydroxy mixed ethers, for example hydroxy mixed ethers of the general formula (V).

The invention is further illustrated by the following, non-limiting working examples.

### Working examples

### Example 1, inventive preparation of (inventive) MGDA granule

### Preparation of Slurry:

202g of Trilon M liquid were heated up to 70°C. Then 98g Trilon M granules were added and stirred and heated for 3h at 70°C. A slurry with a dry content of 58% (42% water) was obtained.

### Preparation of the plates:

Metal plates (rotably mounted) were introduced into a drying cabinet, that was heated to 120°C. The metal plates were warmed up there for 3h.

### Drying:

Then the slurry was put onto the rotably mounted heated metal plates. The metal plates with the slurry on it were introduced into the drying cabinet and dryed for 1h at 120°C. Flakes of dried Trilon M were created and analyzed. These flakes is the inventive MGDA granule in the table below.

### Example 2, inventive preparation of (inventive) co-granules

### Preparation of Slurry:

161,85g of Trilon M liquid were heated up to 70°C. Then 113,2g Trilon M granules and 24,95g Sokalan CP50 liquid (polymer) were added and stirred and heated for 3h at 70°C. A slurry with a dry content of 60% (40% water) was obtained.

For the preparation of the plates and the drying, the instructions from example one were followed.

These flakes, so obtained, is the inventive Co-Granule in the table below.

### Application tests

### Hygroscopicity

### Preparation:

Label each petri dish, then weigh the petri dish empty on the analytical balance and note the tara weight (M0). The samples are well homogenized in advance. Weigh approx. 5 g of the sample on the analytical balance into a petri dish and note the weight of the filled petri dish (M1). The filled petri dishes are placed into the humidity chamber. Remove the samples after 24 h out of the humidity chamber, the weight (M2) is being noted.

| **Hygroscopicity** | storage time: 24h, 35°C/70% rel humidity (Climate Chamber) | | | | |
|---|---|---|---|---|---|
| | | | | | |
| | | | | | |
| Results: | | | | | |

| | **MGDA Granule** | | **Inventive Co-Granule** | | **Inventive MGDA granule** |
|---|---|---|---|---|---|
| | | | | | |
| **after 24h storage** | 24,1 | | 14,2 | | 8,7 |

### Flowability

### Preparation

Weigh approx. 5 g of the sample on the analytical balance into a petri dish and note the weight of the filled petri dish. The filled petri dishes are placed into the humidity chamber. Remove the samples after 1 h, 2 h, 4 h, 6 h, 8 h and 24 h out of the humidity chamber. Each time the samples are taken out of the humidity chamber and the flowability will be checked by visual appearance and slightly shaking of the petri dish. Then put the samples back to the humidity chamber immediately - except after 24 h, the test is finished.

### Results:

| | | | | | |
|---|---|---|---|---|---|
| **Flowability** | storage time: 24h, 35°C/70% rel humidity (Climate Chamber) | | | | |
| | | | | | |
| | | | | | |

| | **MGDA Granule** | | **Inventive Co-Granule** | | **Inventive MGDA granule** |
|---|---|---|---|---|---|
| | | | | | |
| **after 24h storage** | 3,8 | | 1,6 | | 0,4 |
| | | | | | |
| | | **flowability qrades:** | | | |
| | | 0 = good flowable | | | |
| | | 0.5 = good flowable, slightly baked | | | |
| | | 1.0 = slightly baked, flowable when shaking | | | |
| | | 1.5 = slight baked to baked, but flowable when shaking | | | |
| | | 2.0 = baked, not flowable when shaking | | | |
| | | 3.0 = partially solved | | | |
| | | 4.0 = dissolved (no solid form visible) | | | |
| | | | | | |

### Percarbonate stabilization ("yellowing test")

### Preparation:

An amount of 10 g of inventive granule(s) or comparative gr The obtained mixture was filled into a plastic cell bottle* with a semi permeable membrane to allow an exchange with the surrounding atmosphere.anule was mixed with 5 g of sodium percarbonate, 2Na2CO3·3H2O2 (commercially available)e. The vials were stored for 28 days in a climate-chamber at 35°C and 70% humidity. The discoloration - which is a yellowing in this case - of the stored mixtures was determined by measuring the b-value with the CIELAB color space (Mach5 measurement).

### Results:

| **Percarbonate-Stabi lization "Yellowing"** | | | **Measurement: b-value** | | |
|---|---|---|---|---|---|
| | | | | | I |

| | **MGDA Granule** | | **Inventive Co-Granule** | | **inventive MGDA granule** |
|---|---|---|---|---|---|
| Initial | 4,3 | | 2,6 | | 2,3 |
| | | | | | |
| after 28 days | 25,4 | | 19,1 | | 16,7 |

The yellowing/diffuse reflection is determined as b- value.

## Claims

1. Process for making a solid alkali metal salt (A) of an aminocarboxylate complexing agent, said process comprising the steps of
(a) providing a 40 to 80% by weight aqueous solution or slurry, preferably slurry, comprising a salt (A),
(b) introducing the slurry or solution from step (a) into a drying device comprising at least one rotably mounted heated surface for receiving the solution or slurry,
(c) heating said surface to a temperature in the range of from 50° to 250°, preferably in the range from 50° to 220° C, wherein the heating has the effect that the solution or slurry on the rotably mounted surface evaporates and a solid salt (A) remains, and
(d) removing said solid salt (A).

2. Process according to claim 1, wherein the rotably mounted surface in the drying device is heated by a heating fluid, inductively and/or by hot steam.

3. Process according to any of the preceding claims, wherein said aminocarboxylate complexing agent is selected from methylglycine diacetic acid (MGDA), glutamic acid diacetic acid (GLDA) and/or ethylenediamine-N,N'-disuccinic acid (EDDS).

4. Process according to any of the preceding claims, wherein salt (A) is selected from compounds according to general formula (I)
[CH₃-CH(COO)-N(CH₂-COO)₂]M₃₋ₓHₓ (I)
wherein
M is selected from alkali metal cations, same or different, and
x in formula is in the range of from zero to 1.0.

5. Process according to any of the preceding claims, wherein methylglycine diacetate (MGDA) alkali metal salt (A) is selected from the racemic mixture and mixtures of the enantiomers with predominantly the L-enantiomer with an ee value in the range of from 0.1 to 35%.

6. Process according to any of the preceding claims, wherein salt (A) withdrawn in step (d) has a moisture content in the range of from 1 to 30% by weight, preferably 1 to 20% by weight, relative to the total weight of salt (A).

7. Process according to any of the preceding claims, wherein the solution or slurry provided in step (a) contains at least 0.1 weight% of a further organic or inorganic compound or mixtures thereof..

8. Process according to any of the preceding claims, wherein the solution or slurry provided in step (a) contains at least 0.1 weight% of a (co)polymer (B) selected from (co)polymers of (meth)acrylic acid and polyethyleneimines, non-substituted or substituted with alkoxy groups or CH₂COOH groups that may be neutralized with alkali metal.

9. Process according to any of the preceding claims, wherein a sieving step and/or a milling step and/or a compacting step is performed subsequently to step (d), and/or wherein solid alkali metal salt (A) is obtained in the form of a flake, a powder and/or crystal.

10. Process according to any of the preceding claims, wherein the drying device is a disc dryer.

11. Process according to any of the preceding claims, wherein the pressure in the drying device is in the range from 100 to 1100 mbar abs, preferably 100 to 600 mbar abs.

12. Use of a drying device comprising a rotably mounted surface for receiving a solution or slurry, for drying a 40 to 80% by weight aqueous solution or slurry comprising a solid alkali metal salt (A) of an aminocarboxylate complexing agent.

13. Solid alkali metal salt (A) of a mixture of enantiomers of the trisodium salt of MGDA wherein said solid comprises at least 60% by weight of a crystalline material whose lattice has an orthorhombic space group, preferably obtained or obtainable by the process according to any of the preceding claims 1 to 11.

14. Use of a solid alkali metal salt (A) of an aminocarboxylate complexing agent, preferably obtained or obtainable by the process according to any of the preceding claims 1 to 11, for the manufacture of cleaning agents, preferably selected from dishwashing and laundry detergents, more preferably dishwashing detergents, particularly automatic dishwashing detergents.

15. Cleaning agent, preferably selected from dishwashing and laundry detergents, more preferably dishwashing detergents, particularly automatic dishwashing detergents, comprising an aminocarboxylate complexing agent, preferably obtained or obtainable by the process according to any of the preceding claims 1 to 1, and optionally an antimicrobial agent selected from the group consisting of 2-phenoxyethanol; preferably comprising said antimicrobial agent in an amount ranging from 2ppm to 5% by weight of the composition; more preferably comprising 0.1 to 2% of phenoxyethanol.
